# FASCICULE DE BREVET EUROPEEN

(11) **EP 0 932 368 B1**
(45) Date de publication et mention de la délivrance du brevet: **10.09.2003**
(21) Numéro de dépôt: 98924375.3
(22) Date de dépôt: 05.05.1998
(51) Int. Cl.: A61B 17/70

(54) **SYSTEME D'OSTEOSYNTHESE POUR ARTHRODESE VERTEBRALE**
OSTEOSYNTHESESYSTEM FÜR WIRBELARTHRODESE
OSTEOSYNTHESIS SYSTEM FOR VERTEBRA ARTHRODESIS

(30) Priorité: 07.05.1997 FR 9705641
(43) Date de publication de la demande: 04.08.1999
(73) Titulaire: Aesculap AG & Co. KG, 78532 Tuttlingen (DE)
(72) Inventeur: AMEIL, Marc, F-51100 Reims (FR); HUPPERT, Jean, F-42580 l'Etrat (FR); JERMANN, Jean-Louis, F-52901 Chaumont (FR); MARNAY, Thierry, F-34000 Montpellier (FR)
(74) Mandataire: Eidelsberg, Victor Albert
(86) Numéro de dépôt international: FR9800897
(87) Numéro de publication internationale: WO98049960

(56) Documents cités:
- WO-A-97/14368
- DE-C- 4 307 576
- FR-A- 2 693 365
- FR-A- 2 702 361
- US-A- 5 549 608

## Description

La présente invention est relative à la chirurgie d'ostéosynthèse rachidienne et, plus précisément, aux systèmes d'ostéosynthèse pour arthrodèse vertébrale, c'est-à-dire à des systèmes qui sont destinés à immobiliser l'une par rapport à l'autre au moins deux vertèbres adjacentes.

Parmi ces systèmes, l'invention concerne ceux qui comportent au moins une barre de distraction ou de compression vertébrale qui s'étend le long d'une partie au moins du rachis, au moins un, et en général au moins deux, organes d'ancrage vertébral, par exemple une vis pédiculaire ou un crochet, par exemple laminaire, thoracique ou pédiculaire et, associé à chaque organe d'ancrage, un support commun de réception, de couplage et d'immobilisation de ladite barre et d'une extrémité ou tête de l'organe d'ancrage.

Dans de tels systèmes, l'arthrodèse est réalisée du fait de la solidarisation des vertèbres concernées par la barre de distraction ou de compression qui est immobilisée par rapport aux organes d'ancrage par l'intermédiaire des supports.

Parmi ces systèmes, l'invention concerne ceux qui sont du type dit "polyaxial", dans lequel l'organe d'ancrage présente, à l'opposé du rachis, une tête extrême libre à surface sphérique de manière que le support et l'organe d'ancrage puissent avoir une position angulaire relative qui est réglable et modifiable dans toutes les directions en fonction des besoins.

De tels systèmes polyaxiaux sont par exemple décrits dans les demandes de brevet européen 0 553 042 et 0 613 664. Ces systèmes, illustrés par les deux documents ci-dessus cités à titre d'exemple, comportent tous un support creux ou douille dont le fond ouvert présente un logement pour la réception de la tête de l'organe d'ancrage, le corps cylindrique de cette douille présentant deux fentes longitudinales qui débouchent dans l'alésage central pour le passage de la barre de distraction ou de compression transversalement à l'axe de douille. Pour l'immobilisation de l'ensemble, un élément fileté est vissé sur le support et, directement ou indirectement, il agit sur la barre axialement par rapport au support, c'est-à-dire transversalement par rapport à la barre, pour appuyer cette dernière, également directement ou indirectement, contre la tête sphérique de l'organe d'ancrage, cette tête étant ainsi plaquée fermement contre son siège qui est situé dans le fond ouvert du support.

Ces systèmes polyaxiaux connus présentent un inconvénient majeur qui réside en ce que la barre de distraction ou de compression ne peut être mise en place ou enlevée que par déplacement axial par rapport au support, ce qui nécessite entre autres l'enlèvement préalable, et d'une manière générale l'absence, de l'élément fileté d'immobilisation.

Un autre inconvénient de ces systèmes est qu'on ne peut avoir accès à la tête de l'organe sphérique, notamment à l'aide d'un outil, que si la barre et les moyens d'immobilisation sont absents. Cela est très gênant car, d'une part, lors de la mise en place de l'organe d'ancrage sur une vertèbre, par vissage ou par accrochage, la présence de la barre serait utile notamment pour donner au praticien une indication de l'orientation à conférer à l'organe d'ancrage, d'autre part, une fois la barre en place, il est impossible au praticien d'intervenir sur l'organe d'ancrage pour un réglage fin de position du fait que la tête de l'organe d'ancrage est recouverte par la barre et, d'autre part encore, sachant que l'organe d'ancrage doit être enfilé dans le support préalablement à sa mise en place sur le rachis, le support n'est nullement retenu par rapport à l'organe d'ancrage et a donc tendance à flotter ou à tomber le long de la tige de l'organe d'ancrage, ce qui rend au praticien les opérations de mise en place de l'organe d'ancrage très malcommodes.

Un système de ostéosynthèse selon le préambule de la revendication 1 est connue du document FR-A-2 693 365.

L'invention a pour but notamment de remédier à ces inconvénients et, par conséquent, de fournir un système du type ci-dessus dans lequel l'accès de la barre au support et l'accès du praticien à la tête de l'organe d'ancrage sont rendus beaucoup plus commodes.

A cet effet, le système selon l'invention, comporte les caractéristiques de la revendication 1

Ainsi, dans le système selon l'invention, la mise en place de la barre de distraction ou de compression dans son logement, ou son enlèvement de celui-ci, se font par le côté, et plus précisément obliquement du fait que le logement de réception de la barre est ouvert sur le côté et vers le haut, ce qui permet notamment ces opérations alors que l'élément fileté d'immobilisation est déjà ou encore partiellement vissé sur le support, avec pour conséquence une facilité de manoeuvres pour le praticien. Par ailleurs, cette barre n'interfère pas avec la tête de l'organe d'ancrage.

Le premier logement de réception de la barre présente en général une surface concave cylindrique, dont la concavité est orientée à l'opposé du rachis et dont l'axe est normal à l'axe de la partie filetée du support. Pour permettre une bonne coopération de l'écrou avec la barre, la surface qui définit le premier logement coupe de préférence la surface cylindrique de ladite partie filetée du support pour que l'écrou vienne appuyer sur la barre, directement ou indirectement, sensiblement à l'aplomb de l'axe de celle-ci.

Quant à la surface concave du second logement de réception de la tête sphérique de l'organe d'ancrage, elle est avantageusement également sphérique, quoiqu'elle puisse également être conique. La concavité de ce second logement est également orientée à l'opposé du rachis.

Avantageusement, l'écrou peut présenter des crans sur sa face radiale de serrage de la barre, ces crans étant de nature à augmenter la friction et à éviter ainsi un désserrage intempestif de l'écrou.

Pour permettre le réglage angulaire relatif du support et de l'organe d'ancrage, le second logement de réception de la tête sphérique de cet organe est prolongé, vers le rachis, par une ouverture de passage de la tige intermédiaire de l'organe d'ancrage, cette ouverture de passage présentant un diamètre supérieur au diamètre de la tige intermédiaire. De préférence, cette ouverture s'évase vers le rachis et elle est par exemple conique.

De préférence, les moyens filetés d'immobilisation de la barre et de la tête de l'organe d'ancrage sont agencés pour ménager un accès depuis l'extérieur à un profil de manoeuvre de ladite tête, par exemple une empreinte polygonale, lorsqu'ils sont en place sur le support. En général, on fera comporter à ces moyens filetés une ouverture axiale permettant le passage d'un outil.

Suivant un premier mode de réalisation, le second logement de réception de la tête sphérique de l'organe d'ancrage constitue le fond de la partie du support qui est creuse et ouverte axialement de part en part.

Dans ce mode de réalisation, de préférence, les deux logements communiquent latéralement à travers la paroi du support pour que la barre et la tête sphérique s'appuient l'une contre l'autre lors de leur immobilisation ; en d'autres termes, l'entraxe des deux logements est de préférence égal, voire sensiblement inférieur, à la somme des rayons de la barre et de la tête de l'organe d'ancrage. De préférence, les deux logements sont agencés pour que, au montage, l'axe de la barre et le centre de la tête sphérique soient dans un plan transversal perpendiculaire à l'axe de la partie creuse du support.

Suivant un premier mode de mise en oeuvre de ce premier mode de réalisation, on prévoit une vis, de préférence sans tête et noyée, propre à coopérer avec un taraudage intérieur du support creux et présentant une empreinte creuse de manoeuvre, par exemple hexagonale, pour permettre l'immobilisation de la tête sphérique de l'organe d'ancrage dans son logement, c'est-à-dire par rapport au support.

Avantageusement, cette vis est creuse de part en part pour permettre, à travers elle, l'accès d'un outil au profil de manoeuvre de la tête de l'organe d'ancrage.

Quant à l'écrou, il peut coopérer avec la barre soit directement, soit indirectement à l'aide d'une pièce intermédiaire formant rondelle.

Suivant un second mode de mise en oeuvre de ce premier mode de réalisation, on prévoit une pièce intermédiaire constituée d'une partie centrale d'appui sur la tête de l'organe d'ancrage et destinée à être reçue dans l'alésage lisse du support creux, et de deux branches traversant la paroi du support creux par deux fentes longitudinales de celui-ci et de préférence opposées et alignées, l'une des extrémités libres des branches, extérieures au support creux, étant destinée à venir s'appuyer sur la barre, l'ensemble étant tel que l'écrou appuie sur les extrémités libres des deux branches en sollicitant la pièce intermédiaire simultanément contre la tête de l'organe d'ancrage par sa partie centrale et contre la barre par l'extrémité libre de l'une de ses deux branches.

Avantageusement, la partie centrale de la pièce intermédiaire présente une ouverture d'accès au profil de manoeuvre de la tête de l'organe d'ancrage pour un outil.

Par ailleurs, cette pièce intermédiaire est agencée pour s'appuyer par une face simultanément sur la barre et sur la tête de l'organe d'ancrage, de manière à permettre une immobilisation correcte de ces deux éléments par l'écrou.

Suivant un second mode de réalisation de l'invention, le second logement de réception de l'organe d'ancrage est également situé à l'extérieur de la partie filetée du support, lequel est alors de préférence plein dans sa partie filetée, et ce second logement est ouvert latéralement et vers le haut, à l'opposé du rachis, pour que la tête sphérique de l'organe d'ancrage soit également bloquée par l'écrou. Dans ce cas, l'écrou immobilise la barre et la tête de l'organe d'ancrage à l'extérieur du support, par sa partie périphérique, soit par action directe, soit de préférence par l'intermédiaire d'une rondelle.

Cette rondelle présente une ouverture centrale par laquelle elle est enfilée sur la partie filetée du support et qui est prolongée latéralement par une fenêtre, par exemple en arc de cercle, donnant accès au profil de manoeuvre de la tête de l'organe d'ancrage pour un outil.

Dans ce second mode de réalisation, pour obtenir une bonne répartition des efforts et une bonne immobilisation, les deux logements sont de préférence diamétralement opposés par rapport au support et à l'écrou.

Dans tous les modes de réalisation de l'invention, l'écrou peut avantageusement présenter, sur sa face radiale opposée au rachis, des empreintes de manoeuvre destinées à coopérer avec un outil de serrage du genre clef à ergots.

On comprendra bien l'invention à la lecture du complément de description qui va suivre et en référence aux dessins annexés qui font partie de la description et dans lesquels :
Fig. 1 est une vue éclatée en élévation, partiellement en coupe et arrachée, d'un système établi selon un premier mode de réalisation de l'invention, dans son utilisation avec une vis pédiculaire ;
Fig. 2 est une vue en élévation, partiellement en coupe, du système de la Fig. 1 à l'état assemblé ;
Fig. 3 est une vue axiale en plan du support du système des Figs. 1 et 2 ;
Fig. 4 est une vue analogue à la Fig. 2 relativement à l'utilisation d'un organe d'ancrage qui se présente sous la forme d'un crochet ;
Fig. 5 est une vue analogue aux Figs. 2 et 4, relativement à une variante de réalisation des moyens d'immobilisation de la barre et de l'organe d'ancrage par rapport au support, l'organe d'ancrage n'étant représenté que partiellement ;
Fig. 6 est une vue axiale en plan du support du système de la Fig. 5 ;
Fig. 7 est une vue axiale en plan de la pièce intermédiaire de la Fig. 5, située entre, d'une part, l'écrou et, d'autre part, la barre et la tête de l'organe d'ancrage ;
Fig. 8 est une vue analogue aux Figs. 2, 4 et 5, relativement à un second mode de réalisation du système selon l'invention, illustré ici dans son utilisation avec une vis pédiculaire ;
Fig. 9 montre, en coupe axiale, le support du système de la Fig. 8 ;
Fig. 10 est une vue axiale en plan du support de la Fig. 9 ;
Fig. 11 est une vue axiale en plan de la pièce intermédiaire du système de la Fig. 8, cette pièce étant du type rondelle et étant située entre, d'une part, l'écrou de serrage et, d'autre part, la barre et la tête de l'organe d'ancrage ; et
Fig. 12 est une coupe axiale suivant la ligne XII-XII de la pièce intermédiaire de la Fig. 11.

Dans tous les modes de réalisation de l'invention qui vont être décrits ci-dessous à titre d'exemples, le système d'ostéosynthèse pour arthrodèse vertébrale comporte une barre 1 de distraction ou de compression vertébrale, qui est lisse ou qui présente des aspérités, et qui s'étend le long d'une partie au moins du rachis. De place en place, la barre 1 est reçue et immobilisée par des supports qui, chacun, reçoivent et immobilisent un organe d'ancrage vertébral du type polyaxial, par exemple une vis pédiculaire 2 ou un crochet 3 par exemple laminaire, thoracique ou pédiculaire. L'organe d'ancrage 2, 3 comporte, pour sa coopération avec le support, une tête extrême 4 à surface sphérique 5, une tige intermédiaire 6 faisant suite à la tête et une partie d'ancrage vertébral proprement dite qui est constituée soit par une tige filetée 7 dans le cas d'une vis pédiculaire, soit par une partie terminale 8 en crochet dans le cas d'un crochet.

Comme décrit ci-après, la barre 1 et la tête 4 sont reçues par le support et sont couplées et immobilisées sur celui-ci par des moyens filetés qui comportent au moins un écrou 9.

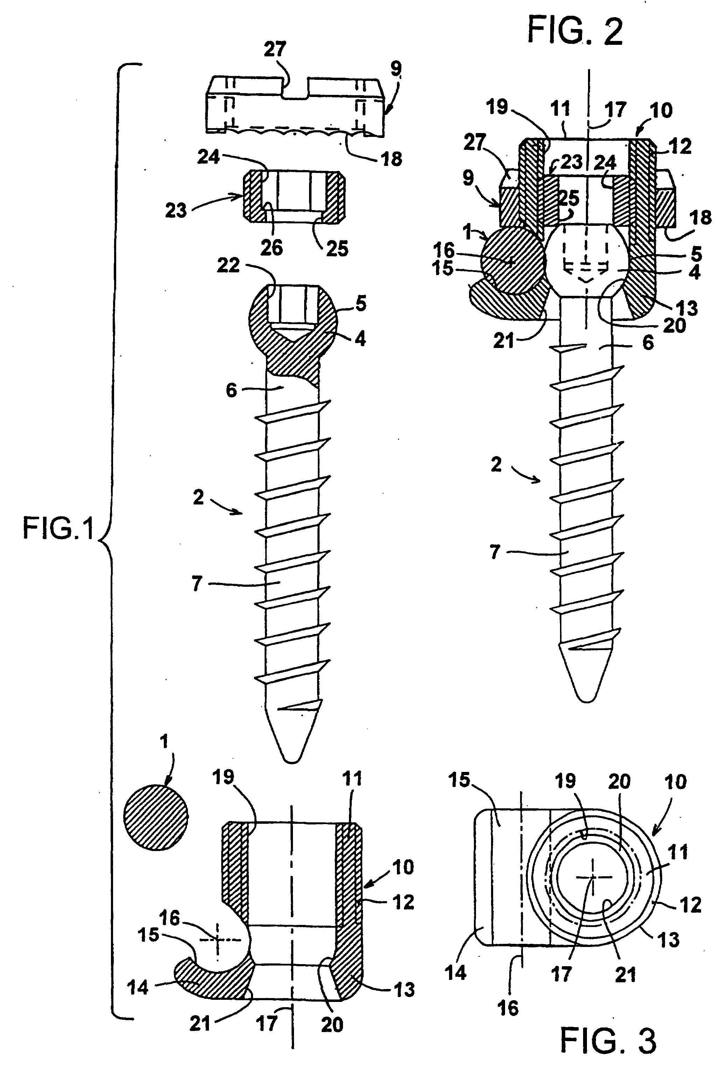

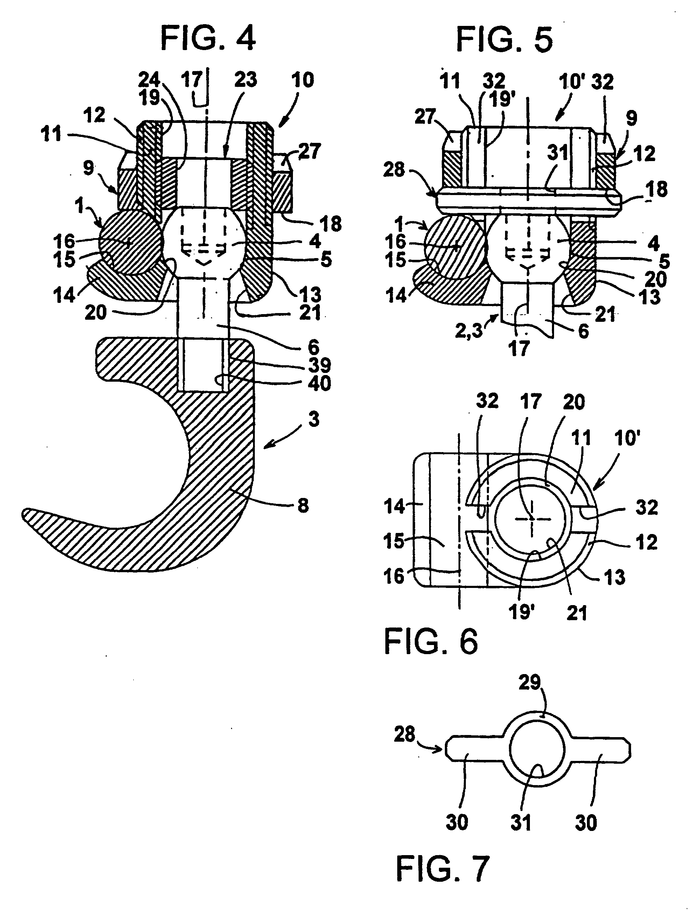

On décrira tout d'abord, en référence aux Figures 1 à 4, un premier mode de réalisation du système selon l'invention, dans son utilisation avec une vis pédiculaire (Figs. 1 à 3) et avec un crochet (Fig. 4).

Le support 10 des Figs. 1 à 4 est constitué par un corps en une seule pièce qui présente une partie principale cylindrique 11 munie sur toute sa longueur, à partir de son extrémité opposée au rachis, c'est-à-dire à partir de son extrémité supérieure sur les Figs. 1, 2 et 4, d'un filetage extérieur 12. La partie principale cylindrique 11 se prolonge, vers le rachis, par une partie cylindrique lisse 13 qui présente une protubérance ou excroissance latérale 14 débordant largement, en projection axiale, du contour circulaire de la partie principale 11.

Dans la protubérance 14 est ménagé un logement concave cylindrique 15, dont la concavité est orientée à l'opposé du rachis et de diamètre sensiblement égal à celui de la barre 1, et de préférence très légèrement inférieur à celui-ci pour obtenir une meilleure coopération faciale entre la barre et son logement. Comme on le voit sur les Figs. 1, 2 et 4, le logement cylindrique 15 est donc situé en partie à l'extérieur de la partie principale cylindrique 11 du support 10, et il est ouvert ou débouche latéralement et vers le haut, c'est-à-dire obliquement, sur l'extérieur, à l'opposé du rachis, ce qui permet une mise en place et un enlèvement de la barre 1 par le côté. L'axe 16 du logement cylindrique 15 est normal à l'axe 17 de la partie cylindrique 11, et il est situé à une distance de celui-ci telle que, comme montré sur les Figs. 2 et 4, l'écrou 9 appuie sur la barre 1 de manière radiale, c'est-à-dire suivant une génératrice à l'aplomb de l'axe commun 16 de la barre et du logement 15 lorsque la barre est en place.

Dans l'exemple représenté, l'écrou 9 appuie directement sur la barre 1 par sa surface radiale inférieure qui, de préférence, comme montré sur la Fig. 1, présente des crans 18 renforçant la friction et de nature à empêcher un désserrage intempestif de l'écrou. En variante, on pourrait prévoir une rondelle intermédiaire entre l'écrou et la barre.

Dans ce mode de réalisation, le support 10 est creux de part en part et il présente successivement et coaxialement, à partir de son extrémité opposée à la protubérance 14, un alésage cylindrique taraudé 19, un logement concave 20 pour la tête sphérique 4 de l'organe d'ancrage, et une ouverture terminale 21 de passage de la tige intermédiaire 6 de l'organe d'ancrage.

Le diamètre de l'alésage taraudé 19 est égal ou sensiblement supérieur à celui de la tête 4 de l'organe d'ancrage, pour permettre l'introduction de celle-ci, tandis que le logement 20 forme un rétrécissement concave pour constituer un siège. Le logement 20, de concavité orientée à l'opposé du rachis, peut être conique ou, de préférence, comme représenté, en forme de partie de sphère dont le rayon est égal à celui de la tête sphérique 4, de manière à fournir une articulation du type à rotule. L'ouverture terminale 21 s'évase vers le bas, c'est-à-dire vers le rachis, par exemple en cône, à partir de l'extrémité du logement 20 opposée à l'alésage taraudé 19, pour permettre un réglage angulaire de la position relative entre le support 10 et l'organe d'ancrage 2,3. A sa jonction avec le logement 20, l'ouverture 21 présente, dans le même but et comme montré sur les Figs. 2 et 4, un diamètre qui est supérieur à celui de la tige intermédiaire 6 de l'organe d'ancrage.

Suivant une caractéristique avantageuse, également visible sur les dessins, les logements 15 et 20 communiquent latéralement l'un avec l'autre, à travers la paroi du support 10 pour que, lors du serrage, la barre 1 et la tête sphérique 4 de l'organe d'ancrage s'appuient l'une contre l'autre, ce qui renforce l'immobilisation de l'ensemble. Pour cela, la plus courte distance entre les axes 16 et 17 est égale ou de préférence légèrement inférieure à la somme des rayons de la barre 1 et de la tête 4. Par ailleurs, les logements 15, 20 sont agencés pour que, au montage, l'axe commun 16 de la barre 1 et le centre de la tête 4 soient à la même hauteur, c'est-à-dire dans un plan transversal perpendiculaire à l'axe 17 du support 10.

Pour son serrage dans le corps vertébral, la tête sphérique 4 de l'organe d'ancrage 2,3 présente une empreinte en creux 22, par exemple polygonale, et notamment hexagonale, destinée à recevoir un outil mâle de profil complémentaire.

Pour l'immobilisation de la tête 4 par rapport au support 10, on prévoit une vis 23 destinée à coopérer avec l'alésage taraudé 19 du support 10 et présentant une empreinte creuse 24, par exemple également polygonale, et notamment hexagonale, pour la réception d'un outil mâle de profil complémentaire. Suivant une caractéristique avantageuse, la vis 23 est creuse de part en part pour permettre l'accès du premier outil à l'empreinte 22 de la tête 4 et, ainsi, une action manuelle extérieure sur l'organe d'ancrage alors que la vis de blocage 23 est déjà en place. Pour cela, l'empreinte 24 est plus grande que l'empreinte 22 et elle est prolongée de préférence par un alésage cylindrique 25 de diamètre au moins égal au diamètre du cercle circonscrit de l'empreinte 22 et définissant au surplus un épaulement 26 de butée axiale pour l'outil de manoeuvre de la vis 23. Comme on le voit sur les dessins, la vis 23 est sans tête, ce qui lui permet d'être totalement noyée dans le support 10 lors du serrage (Figs. 2 et 4). Lors du serrage, elle appuie sur la tête 4 le long du bord inférieur circulaire de l'alésage 25.

Pour la manoeuvre de l'écrou 9, on peut faire comporter à celui-ci, sur sa face radiale opposée aux crans 18, des empreintes en creux 27 pour la coopération avec un outil complémentaire à ergots.

Dans le mode de réalisation des Figs 1 à 4 qui vient d'être décrit, les moyens filetés d'immobilisation de la barre 1 et de l'organe d'ancrage 2,3 sur le support 10 sont constitués par deux éléments distincts et à manoeuvre indépendante, à savoir l'écrou 9 pour la barre 1 et la vis 23 pour l'organe d'ancrage.

Lorsque l'organe d'ancrage 2,3 est porté par le support 10, la vis 23 peut être vissée, mais sans serrer la tête 4, pour qu'il existe un faible jeu entre le support et la tête, ce qui permet de mettre en place l'organe d'ancrage sur le rachis, par action à travers la vis 23, sans que le support 10 ne flotte ou ne tombe le long de la tige intermédiaire 6. Lors de cette mise en place de l'organe d'ancrage, l'écrou 9 peut, sans gêner, être présent sur son filetage 12, et la barre 1 peut également être présente dans son logement 15, sans que l'écrou 9 soit complètement serré ; cela permet au praticien, avant le blocage final par l'écrou 9 et la vis 23, d'ajuster finement les positions relatives de l'organe d'ancrage 2,3, du support 10 et de la barre 1.

Au surplus, après ce blocage final, le praticien peut encore ajuster, de manière indépendante et sélective, la position relative du support 10 et de la barre 1 et/ou de la tête 4 par un léger desserrage respectivement de l'écrou 9 et/ou de la vis 23, suivi de l'ajustement de position désiré et d'un resserrage.

On décrira maintenant, en référence aux Figs. 5 à 7, une variante du mode de réalisation des Figs. 1 à 4, cette variante étant bien évidemment applicable à une utilisation aussi bien avec la vis pédiculaire 2 qu'avec le crochet 3.

Cette variante se caractérise essentiellement en ce que l'immobilisation de la barre 1 et de la tête sphérique 4 de l'organe d'ancrage 2,3 se fait uniquement par l'écrou 9, à l'aide d'une pièce intermédiaire 28 qui coopère, d'une part, avec l'écrou 9 et, d'autre part, avec la barre 1 et la tête sphérique 4.

Dans sa partie inférieure de réception de la barre 1 et de la tête sphérique 4, le support 10' est identique ou similaire au support 10. On pourra donc se référer à ce sujet à la description qui a été faite en relation avec le support 10. A sa partie supérieure, le support 10' comporte également une partie principale cylindrique 11 qui est filetée en 12 pour recevoir l'écrou 9, qui est également identique à celui des Figs. 1 à 4.

Le support 10' est également creux de part en part et, à sa partie supérieure, c'est-à-dire au-dessus du logement 20, il présente un alésage cylindrique lisse 19' pour le passage de la tête sphérique 4 et, comme décrit ci-après, de la partie centrale de la pièce intermédiaire 28.

La pièce intermédiaire 28 comporte une partie centrale 29 et deux branches 30 faisant saillie vers l'extérieur à partir de la partie centrale 29 et de préférence opposées et alignées. La partie centrale 29 est circulaire, d'un diamètre extérieur égal ou à peine inférieur au diamètre de l'alésage cylindrique 19', et elle présente une ouverture circulaire centrale 31 permettant l'accès de l'outil de serrage à l'empreinte de manoeuvre de la tête sphérique 4. Les branches radiales 30 de la pièce intermédiaire 28 traversent la paroi de la partie cylindrique 11 du support 10' par deux fentes longitudinales 32 qui s'étendent axialement sur une distance telle que la pièce intermédiaire 28 puisse venir s'appuyer par sa partie centrale 29 sur la tête sphérique 4 et par l'extrémité de l'une de ses branches 30 sur la barre 1, à l'aplomb de l'axe 16 du logement 15. Par conséquent, les branches 30, à partir de la partie centrale 29, traversent la paroi du support 10' pour que leurs extrémités libres soient situées à l'extérieur du support de manière à coopérer, par une face, avec l'écrou 9 et, par l'autre face pour l'une seulement des branches 30, avec la barre 1.

Lors du serrage, l'écrou 9 appuie sur les extrémités libres diamétralement opposées des branches 30, ce qui sollicite la partie centrale 29 contre la tête sphérique de l'organe d'ancrage, par le bord inférieur circulaire de l'ouverture 31, à l'extérieur de l'empreinte de la tête 4, et l'extrémité libre de l'une des branches 30 contre la barre.

Dans ce mode de réalisation, la fente longitudinale 32 qui est située du côté du logement 15 peut être totalement ouverte à sa partie inférieure, pour déboucher dans le logement 15 de la barre 1. Comme montré sur la Fig. 6, le plan axial des fentes 32 est perpendiculaire à l'axe 16 du logement de barre 15.

Lors de la mise en place de l'organe d'ancrage 2,3 sur le rachis, l'écrou 9 et la pièce intermédiaire 28 sont de préférence déjà présents sur le support 10', mais sans serrage de la tête 4 pour permettre un léger jeu entre la tête et le support. Cela évite au support de trop flotter ou de tomber le long de la tige intermédiaire. Au surplus, la barre 1 peut également être présente dans son logement 15, sans serrage et sans gêner l'action du praticien sur la tête 4. Au contraire, la présence simultanée de la barre 1, du support 10' et de l'organe d'ancrage 2,3 permet au praticien, avant le blocage final par l'écrou 9, d'ajuster finement la position relative de ces éléments.

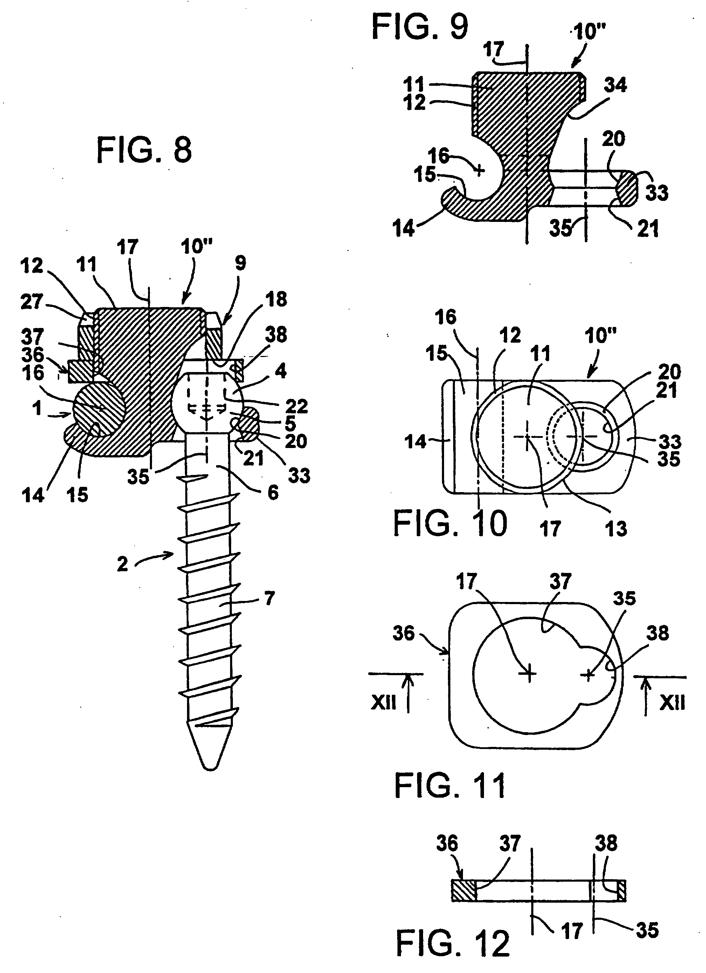

On décrira maintenant, en référence aux Figs. 8 à 12, le second mode de réalisation du système selon l'invention.

Ce second mode de réalisation diffère du précédent essentiellement en ce que, comme décrit ci-après, le logement de réception de la tête sphérique de l'organe d'ancrage est également situé à l'extérieur de la partie filetée du support et est ouvert latéralement et vers le haut pour que la tête sphérique de l'organe d'ancrage soit également bloquée par l'écrou et que ce second logement soit accessible par le côté, et non plus axialement par l'intérieur du corps du support.

A sa partie inférieure, le support 10" comporte, comme précédemment, la protubérance 14 définissant le logement 15 de réception de la barre 1, l'agencement géométrique de ce logement 15 étant le même que précédemment, de sorte qu'on pourra se référer à la description précédente.

Dans ce mode de réalisation, la partie principale cylindrique 11 du support 10" est pleine, c'est-à-dire non alésée, et elle comporte extérieurement, comme précédemment, le filetage 12 pour la réception de l'écrou 9 qui est également identique ou similaire à l'écrou 9 qui a été décrit ci-dessus.

A sa partie inférieure, et à l'opposé de la protubérance 14, le corps du support 10" comporte une protubérance ou excroissance latérale 33, surmontée d'une large échancrure 34 qui est pratiquée sur la partie cylindrique 11 du corps du support 10" et qui va en s'amincissant vers le haut. La protubérance 33 est creuse de part en part et son alésage est constitué par un logement 20 et par une ouverture de passage 21 qui sont identiques à ceux qui ont été décrits précédemment, respectivement pour la réception de la tête cylindrique 4 de l'organe d'ancrage, représenté ici à titre d'exemple par la vis pédiculaire 2, et pour le passage de la tige intermédiaire 6 de cet organe. L'axe 35 de l'alésage 20, 21 est parallèle à l'axe 17 de la partie principale cylindrique 11 du support, et il est situé, de préférence, sensiblement à la même distance de celui-ci que l'axe 16 du logement 15 pour la barre 1. Du fait que les protubérances 14 et 33 sont opposées, le plan des axes 17 et 35 est perpendiculaire à l'axe 16 du logement de barre 15.

Le serrage de la barre 1 et de la tête sphérique 4 par l'écrou 9 se fait à l'aide d'une pièce intermédiaire 36 du type rondelle. La rondelle 36 présente une ouverture centrale circulaire 37 pour le passage de la partie cylindrique 11 du support, cette ouverture 37 étant prolongée, du côté de l'alésage 20, 21, par une fenêtre 38 en arc de cercle, coaxiale à l'alésage 20, 21 pour que, comme montré sur la Fig. 8, son bord inférieur en arc de cercle vienne coopérer avec la tête sphérique de l'organe d'ancrage lors du serrage, à l'extérieur de l'empreinte 22. Par ailleurs, cette fenêtre 38 permet un accès de l'outil à la tête de l'organe d'ancrage, notamment dans le cas d'une vis pédiculaire. L'échancrure 34 permet le passage de l'organe d'ancrage et de l'outil de manoeuvre de celui-ci.

Lors du serrage de l'écrou 9, la rondelle 36 vient s'appuyer suivant deux zones diamétralement opposées respectivement contre la barre 1 et la tête sphérique 4 de l'organe d'ancrage, ce qui assure une bonne répartition des efforts et un excellent blocage.

Dans ce mode de réalisation, l'écrou 9 ne peut pas être présent sur le support 10", lors de la mise en place de l'organe d'ancrage sur le rachis, du fait que l'empreinte 22 est à l'aplomb du filetage 12. Le support 10" n'est donc pas retenu.

En revanche, ce mode de réalisation présente le même avantage lié à la possibilité d'accéder à l'organe d'ancrage alors que la barre 1 est en place, mais avec la nécessité d'enlever l'écrou 9.

Dans les divers modes de réalisation de l'invention, la tête sphérique 4, dans le cas de la vis pédiculaire 2, présente un diamètre en général supérieur à celui de la tige intermédiaire 6 et de la tige filetée 7, de sorte que la vis pédiculaire peut être fabriquée en une seule pièce et être enfilée dans le support 10, 10' ou 10". En revanche, si le diamètre de la tête sphérique 4 est inférieur à l'un ou l'autre des diamètres de la tige intermédiaire 6 et de la tige filetée 7, une telle mise en place axiale n'est pas possible et, dans ce cas, la vis pédiculaire sera fabriquée en deux pièces qui seront assemblées après la mise en place de la tête sphérique 4 dans son logement 20. Cela s'applique également au cas où l'organe d'ancrage est constitué par le crochet 3 représenté sur la Fig. 4 et utilisable dans tous les modes de réalisation de l'invention. Dans ce cas, l'extrémité libre de la tige intermédiaire 6 porte un filetage 39 coopérant avec un taraudage 40 de la partie 8 en crochet.

Bien entendu, l'invention n'est pas limitée aux modes de réalisation qui ont été décrits ; on pourrait au contraire concevoir diverses variantes sans sortir pour autant de son cadre.

## Revendications

1. Système d'ostéosynthèse pour arthrodèse vertébrale, comportant : au moins une barre (1) de distraction ou de compression vertébrale propre à s'étendre sur une partie au moins du rachis ; au moins un organe d'ancrage vertébral orientable (2, 3) comprenant une tête (4) à surface sphérique (5), et une partie d'ancrage vertébral (7, 8) ; la tête sphérique (4, 5) de l'organe d'ancrage (2, 3) constitue l'extrémité de celui-ci opposée à la partie d'ancrage (7, 9); un support commun (10, 10', 10") de réception, de couplage et d'immobilisation dudit organe d'ancrage vertébral et de ladite barre, ce support commun comprenant un premier logement concave (15) de réception de la barre (1), et étant ourert vers le haut, à l'opposé de ladite partie d'ancrage, et un second logement concave (20) de réception de ladite tête sphérique (4), ce second logement étant agencé pour que la tête sphérique puisse occuper par rapport au support toute position angulaire réglable autour de son centre, le premier logement étant décalé latéralement par rapport à ce second logement ; et des moyens filetés (9, 23) d'immobilisation de la barre et de l'organe d'ancrage sur le support, ces moyens d'immobilisation comprenant au moins un écrou (9), **caractérisé en ce que** : l'écrou (9) se vissant sur une partie filetée (11, 12); ladite partie filetée (11, 12) appartient au support (10, 10', 10"); au moins une partie du premier logement (15) est située latéralement à l'extérieur de ladite partie filetée ; ce premier logement étant ouvert latéralement et la concavité des deux logements (15, 20) est tournée axialement à l'opposé de la partie d'ancrage (7, 8) de l'organe d'ancrage, l'ensemble étant tel que, lorsque l'écrou (9) est vissé sur la partie filetée (11, 12) du support, (10, 10', 10") il immobilise axialement la barre (1) dans son logement.

2. Système selon la revendication 1, **caractérisé en ce que** le premier logement (15) présente une surface concave cylindrique dont la concavité est orientée à l'opposé du rachis et dont l'axe (16) est normal à l'axe (17) de la partie filetée (11, 12) du support.

3. Système selon l'une des revendications 1 et 2, **caractérisé en ce que** la surface définissant le premier logement (15) coupe la surface cylindrique de la partie filetée (11, 12) du support.

4. Système selon l'une des revendications 1 à 3, **caractérisé en ce que** la surface concave du second logement (20) est sphérique ou conique et la concavité de cette surface est orientée à l'opposé du rachis.

5. Système selon l'une des revendications 1 à 4, **caractérisé en ce que** l'écrou (9) présente des crans (18) sur sa surface radiale de serrage de la barre (1).

6. Système selon l'une des revendications 1 à 5, **caractérisé en ce que** le second logement (20) est prolongé vers le rachis par une ouverture (21) de passage d'une tige intermédiaire (6) de l'organe d'ancrage située entre la tête sphérique (4) et la partie d'ancrage (7, 8) de celui-ci.

7. Système selon la revendication 6, **caractérisé en ce que** l'ouverture (21) de passage de la tige intermédiaire (6) présente un diamètre supérieur au diamètre de ladite tige intermédiaire pour permettre un réglage de la position angulaire de l'organe d'ancrage par rapport au support dans toutes les directions.

8. Système selon la revendication 7, **caractérisé en ce que** l'ouverture de passage (21) s'évase vers le rachis et est de préférence conique.

9. Système selon l'une des revendications 1 à 8, **caractérisé en ce que** le second logement (20) constitue le fond de la partie creuse et ouverte (11, 12) du support.

10. Système selon la revendication 9, **caractérisé en ce que** le premier et le second logements communiquent latéralement l'un avec l'autre à travers la paroi du support, pour que la barre et la tête sphérique s'appuient l'une contre l'autre lors de leur immobilisation, la distance entre les axes des deux logements étant égale ou légèrement inférieure à la somme des rayons de la barre et de la tête sphérique.

11. Système selon l'une des revendications 9 et 10, **caractérisé en ce qu'**il comporte une vis (23), de préférence sans tête et noyée, propre à coopérer avec un taraudage intérieur (19) du support creux (10) et présentant une empreinte creuse (24) de manoeuvre, par exemple polygonale, et notamment hexagonale, pour permettre l'immobilisation de la tête sphérique de l'organe d'ancrage dans le second logement (20).

12. Système selon la revendication 11, **caractérisé en ce que** la vis (23) est creuse de part en part pour permettre l'accès d'un outil à un profil de manoeuvre (22) ménagé dans la tête sphérique de l'organe d'ancrage.

13. Système selon l'une des revendications 1 à 12, **caractérisé en ce que** l'écrou (9) coopère directement avec la barre (1).

14. Système selon l'une des revendications 9 et 10, **caractérisé en ce qu'**il comporte une pièce intermédiaire (28) constituée d'une partie centrale (29) d'appui sur la tête sphérique (4) de l'organe d'ancrage et destinée à être reçue dans un alésage lisse (19') du support creux (10'), et de deux branches (30) traversant la paroi du support creux par deux fentes longitudinales (32) de celui-ci et de préférence opposées et alignées, l'une des extrémités libres des branches (30), extérieures au support creux, étant destinée à venir s'appuyer sur la barre (1), l'ensemble étant tel que l'écrou (9) appuie sur les extrémités libres des deux branches (30) en sollicitant la pièce intermédiaire (28) simultanément contre la tête sphérique (4) de l'organe d'ancrage par sa partie centrale (29) et contre la barre (1) par l'extrémité libre de l'une de ses deux branches (30).

15. Système selon la revendication 14, **caractérisé en ce que** la partie centrale (29) de la pièce intermédiaire (28) présente une ouverture (31) dont le bord est destiné à venir s'appuyer suivant un cercle sur la tête sphérique de l'organe d'ancrage et qui permet l'accès d'un outil à un profil de manoeuvre ménagé dans ladite tête sphérique.

16. Système selon l'une des revendications 1 à 8, **caractérisé en ce que** le second logement (20) est également situé à l'extérieur de la partie filetée (11, 12) du support (10"), qui est de préférence plein dans sa partie filetée, et est ouvert latéralement et vers le haut, à l'opposé du rachis, pour que la tête sphérique (4) de l'organe d'ancrage soit également bloquée par l'écrou (9), à l'extérieur du support (10").

17. Système selon la revendication 16, **caractérisé en ce que** l'écrou (9) agit sur la barre (1) et sur la tête sphérique (4) par l'intermédiaire d'une rondelle (36).

18. Système selon l'une des revendications 16 et 17, **caractérisé en ce que** les premier et second logements (15, 20) sont diamétralement opposés par rapport au support et à l'écrou.

19. Système selon l'une des revendications 17 et 18, **caractérisé en ce que** la rondelle (36) présente une fenêtre (38) dont le bord est destiné à coopérer suivant un arc de cercle avec la tête sphérique (4) et qui permet l'accès d'un outil à un profil de manoeuvre porté par la tête sphérique.

20. Système selon l'une des revendications 1 à 19, **caractérisé en ce que** l'écrou (9), sur sa face radiale opposée au rachis, présente des empreintes de manoeuvre (27) destinées à coopérer avec un outil de serrage.

21. Système selon l'une des revendications 1 à 20, **caractérisé en ce que** l'organe d'ancrage appartient au groupe constitué par des vis pédiculaires (2) et des crochets (3), par exemple laminaires, thoraciques ou pédiculaires.

22. Système selon l'une des revendications 1 à 21, **caractérisé en ce que** les moyens filetés d'immobilisation de la barre (1) et de la tête (4) de l'organe d'ancrage sont agencés pour ménager un accès depuis l'extérieur à un profil de manoeuvre porté par ladite tête, notamment par une ouverture.

## Patentansprüche

1. System zur Osteosynthese für eine vertebrale Arthrodese, mit:
mindestens einem Stab (1) zur vertebralen Dehnung oder Komprimierung, der geeignet ist, sich auf mindestens einen Teil der Wirbelsäule zu erstrecken;
mindestens ein ausrichtbares vertebrales Verankerungselement (2,3) mit einem Kopf (4) mit kugelförmiger Oberfläche (5), und einem vertebralen Verankerungsteil (7,8);
wobei der kugelförmige Kopf (4,51) des Verankerungselements (2,3) dessen dem Verankerungsteil (7,8) entgegengesetztes Ende bildet;
einem gemeinsamen Träger (10,10',10") zur Aufnahme, zum Koppeln und zum Feststellen des vertebralen Verankerungselements und des Stabs, wobei dieser gemeinsame Träger einen ersten konkaven Sitz (15) zur Aufnahme des Stabs (1) umfasst und nach oben gegenüber dem Verankerungsteil offen ist, sowie einen zweiten konkaven Sitz (20) zur Aufnahme des Kugelkopfs (4), wobei dieser zweite Sitz so angeordnet ist, dass der Kugelkopf im Verhältnis zum Träger jede um seine Mitte regelbare Winkelposition einnehmen kann, wobei der erste Sitz seitlich in Bezug auf den zweiten Sitz verschoben ist;
und mit Gewinde versehene Mittel (9,23) zum Feststellen des Stabs und des Verankerungselements am Träger, wobei diese Feststellmittel mindestens eine Mutter (9) aufweisen, **dadurch gekennzeichnet, dass** die Mutter (9) an einem Gewindeteil (11,12) angeschraubt ist/wird, der Gewindeteil (11,12) zum Träger (10,10',10") gehört, mindestens ein Teil des ersten Sitzes (15) lateral außerhalb des Gewindeteils gelegen ist, wobei dieser erste Sitz seitlich offen ist, und die konkave Ausnehmung der beiden Sitze (15,20) axial entgegengesetzt zum Verankerungsteil (7,8) des Verankerungselements gerichtet ist, wobei die Einheit so aufgebaut ist, dass die Mutter (9), wenn sie auf den Gewindeteil (11,12) des Trägers (10,10',10") geschraubt wird, den Stab (1) in seinem Sitz axial feststellt.

2. System nach Anspruch 1, **dadurch gekennzeichnet, dass** der erste Sitz (15) eine zylindrische konkave Oberfläche aufweist, deren Konkavität entgegengesetzt zu der Wirbelsäule orientiert ist und deren Achse (16) normal bzw. senkrecht zur Achse (17) des Gewindeteils (11,12) des Trägers ist.

3. System nach einem der Ansprüche 1 und 2, **dadurch gekennzeichnet, dass** die den ersten Sitz (15) festlegende Oberfläche die zylindrische Oberfläche des Gewindeteils (11,12) des Trägers schneidet.

4. System nach einem der Ansprüche 1 bis 3, **dadurch gekennzeichnet, dass** die konkave Oberfläche des zweiten Sitzes (20) kugelförmig oder konisch ist und die Konkavität dieser Oberfläche entgegengesetzt zur Wirbelsäule orientiert ist.

5. System nach einem der Ansprüche 1 bis 4, **dadurch gekennzeichnet, dass** die Mutter (9) Rasten bzw. Kerben (18) auf ihrer Radialfläche zum Festziehen des Stabs (1) aufweist.

6. System nach einem der Ansprüche 1 bis 5, **dadurch gekennzeichnet, dass** der zweite Sitz (20) zur Wirbelsäule hin durch eine Öffnung (21) zum Durchgang einer Zwischenstange (6) des Verankerungselements, die zwischen dem Kugelkopf (4) und dem Verankerungsteil (7,8) desselben gelegen ist, verlängert ist.

7. System nach Anspruch 6, **dadurch gekennzeichnet, dass** die Öffnung (21) zum Durchgang der Zwischenstange (6) einen größeren Durchmesser aufweist als der Durchmesser der Zwischenstange, um eine Regelung der Winkelposition des Verankerungselements in Bezug auf den Träger in allen Richtungen zu ermöglichen.

8. System nach Anspruch 7, **dadurch gekennzeichnet, dass** die Öffnung des Durchgangs (21) sich zur Wirbelsäule hin ausweitet und vorzugsweise konisch ist.

9. System nach einem der Ansprüche 1 bis 8, **dadurch gekennzeichnet, dass** der zweite Sitz (20) den Boden des hohlen und offenen Teils (11,12) des Trägers bildet.

10. System nach Anspruch 9, **dadurch gekennzeichnet, dass** der erste und zweite Sitz lateral über die Wand des Trägers miteinander in Verbindung stehen, damit der Stab und der Kugelkopf bei ihrer Feststellung sich gegenseitig abstützen, wobei der Abstand zwischen den Achsen der beiden Sitze gleich oder geringfügig kleiner ist als die Summe der Radien des Stabs und des Kugelkopfs.

11. System nach einem der Ansprüche 9 und 10, **dadurch gekennzeichnet, dass** er eine Schraube (23), vorzugsweise ohne Kopf und versenkt, umfasst, die geeignet ist, mit einem Innengewinde (19) des hohlen Trägers (10) zusammenzuwirken und eine hohle, beispielsweise polygonale und insbesondere sechseckige Handhabungs-Ausnehmung aufweist, um die Feststellung des kugelförmigen Kopfs des Verankerungselements im zweiten Sitz (20) zu ermöglichen.

12. System nach Anspruch 11, **dadurch gekennzeichnet, dass** die Schraube (23) durchgehend hohl ist, um den Zugang eines Werkzeugs zu einem Handhabungsprofil (22) zu ermöglichen, das im Kugelkopf des Verankerungselements angeordnet ist.

13. System nach einem der Ansprüche 1 bis 12, **dadurch gekennzeichnet, dass** die Mutter (9) direkt mit dem Stab (11) zusammenwirkt.

14. System nach einem der Ansprüche 9 und 10, **dadurch gekennzeichnet, dass** es ein Zwischenstück (28) umfasst, das aus einem zentralen Teil (29) zum Abstützen am Kugelkopf (4) des Verankerungselements besteht und dazu vorgesehen ist, in einer glatten Bohrung (19') des hohlen Trägers (10') aufgenommen zu werden, sowie zwei Zweige (30), welche die Wand des hohlen Trägers über zwei Longitudinalschlitze (32) durchsetzen und vorzugsweise einander gegenüberliegen und zueinander ausgerichtet sind, wobei eines der freien Enden der Zweige (30) außerhalb des hohlen Trägers dazu vorgesehen ist, sich an dem Stab (1) abzustützen, wobei die Einheit so aufgebaut ist, dass sich die Mutter (9) an den freien Enden der Zweige (30) abstützt, indem das Zwischenstück (28) gleichzeitig gegen den Kugelkopf (4) des Verankerungselements durch seinen mittleren Teil (29) und gegen den Stab (1) durch das freie Ende eines seiner zwei Zweige (30) vorbelastet ist.

15. System nach Anspruch 14, **dadurch gekennzeichnet, dass** der mittlere Teil (29) des Zwischenstücks (28) eine Öffnung (31) aufweist, deren Rand dazu vorgesehen ist, sich einer Kreisform folgend am Kugelkopf des Verankerungselements abzustützen, und der den Zugang eines Werkzeugs zu einem in dem Kugelkopf angeordneten Handhabungsprofil ermöglicht.

16. System nach einem der Ansprüche 1 bis 8, **dadurch gekennzeichnet, dass** der zweite Sitz (20) ebenfalls außerhalb des Gewindeteils (11,12) des Trägers (10") gelegen ist, der vorzugsweise in seinem Gewindeteil voll bzw. massiv ist und nach oben entgegengesetzt zur Wirbelsäule seitlich geöffnet ist, damit der Kugelkopf (4) des Verankerungselements ebenfalls durch die Mutter (9) außerhalb des Trägers (10") verblockt ist.

17. System nach Anspruch 16, **dadurch gekennzeichnet, dass** die Mutter (9) auf den Stab (1) und über eine Scheibe (36)auf den Kugelkopf (4) einwirkt.

18. System nach einem der Ansprüche 16 und 17, **dadurch gekennzeichnet, dass** die ersten und zweiten Sitze (15,20) in Bezug auf den Träger und die Mutter diametral entgegengesetzt sind.

19. System nach einem der Ansprüche 17 und 18, **dadurch gekennzeichnet, dass** die Scheibe (36) ein Fenster (38) aufweist, dessen Rand dazu vorgesehen ist, gemäß einem Kreisbogen mit dem Kugelkopf (4) zusammenzuwirken, und das den Zugang eines Werkzeugs zu einem von dem Kugelkopf getragenen Handhabungsprofil ermöglicht.

20. System nach einem der Ansprüche 1 bis 19, **dadurch gekennzeichnet, dass** die Mutter (9) auf ihrer der Wirbelsäule entgegengesetzten Radialfläche Handhabungs-Ausnehmungen (27) aufweist, die dazu vorgesehen sind, mit einem Befestigungswerkzeug zusammenzuwirken.

21. System nach einem der Ansprüche 1 bis 20, **dadurch gekennzeichnet, dass** das Verankerungselement zu der Gruppe gehört, die aus pedikulären Schrauben (2) sowie Haken (3), beispielsweise laminaren, thoraxartigen oder pedikulären, gebildet ist.

22. System nach einem der Ansprüche 1 bis 21, **dadurch gekennzeichnet, dass** die Gewindemittel zum Feststellen des Stabs (1) und des Kopfs (4) des Verankerungselements so angeordnet sind, dass sie einen Zugang von außen zu einem von dem Kopf getragenen Handhabungsprofil, insbesondere über eine Öffnung, schaffen.

## Claims

1. An osteosynthesis system for vertebral arthrodesis, comprising : at least one vertebral compression or distraction bar (1) capable of extending over a portion at least of the rachis ; at least one orientable vertebral anchoring member (2, 3) comprising a head (4) having a spherical surface (5), and a vertebral anchoring portion (7, 8) ; the spherical head (4, 5) of the anchoring member (2, 3) constitutes the end of the anchoring member, that is opposite to the anchoring portion ; a common support (10, 10', 10") for receiving, coupling and immobilising said vertebral anchoring member and said bar, said common support comprising a first concave housing (15) for receiving the bar, and being open upwardly, opposite to said anchoring portion, and a second concave housing (20) for receiving said spherical head (4), said second housing being so arranged that the said spherical head can occupy with respect to the said support any regulatable angular position about its center, said first housing being laterally offset with respect to said second housing ; and screwthreaded means (9, 23) for immobilisation of the bar and the anchoring member on the support, said immobilisation means comprising at least one nut (9) **characterized in that** said nut (9) is screwed onto a screwthreaded portion (11, 12), said screwthreaded portion (11, 12) belongs to the support (10, 10', 10"); at least a portion of the first housing (15) is disposed laterally to the exterior of said screwthreaded portion, said first housing being open laterally ; and the concavity of the two housing (15, 20) is directed axially in opposite relationship to the anchoring portion (7, 8) of the anchoring member, the whole assembly being such that, when the nut (9) is screwed onto the screwthreaded portion (11, 12) of the support (10, 10', 10"), it axially immobilises the bar (1) in its housing.

2. A system according to claim 1 **characterized in that** the first housing (15) has a cylindrical concave surface whose concavity is oriented in opposite relationship to the rachis and of which the axis (16) is normal to the axis (17) of the screwthreaded portion (11, 12) of the support.

3. A system according to claim 1 or 2 **characterized in that** the surface defining the first housing (15) intersects the cylindrical surface of the screwthreaded portion (11, 12) of the support.

4. A system according to one of claims 1 to 3, **characterized in that** the concave surface of the second housing (20) is spherical or conical and the concavity of that surface is oriented in opposite relationship to the rachis.

5. A system according to one of claims 1 to 4, **characterized in that** the nut (9) has teeth (18) on its radial surface for clamping the bar (1).

6. A system according to one of claims 1 to 5, **characterized in that** the second housing (20) is prolonged towards the rachis by an opening (21) for the passage of an intermediate shank (6) of the anchoring member, said shank being disposed between the spherical head (4) and the anchoring portion (7, 8) of the anchoring member.

7. A system according to claim 6, **characterized in that** the opening (21) for the passage of the intermediate shank (6) is of a diameter which is larger than the diameter of said intermediate shank to permit adjustment of the angular position of the anchoring member with respect to the support in all directions.

8. A system according to claim 7, **characterized in that** the passage opening (21) flares towards the rachis and is preferably conical.

9. A system according to one of claims 1 to 8, **characterized in that** the second housing (20) constitutes the bottom of the hollow open portion (11, 12) of the support.

10. A system according to claim 9, **characterized in that** the first and second housings communicate laterally with each other through a wall of the support so that the bar and the spherical head bear against each other upon immobilisation thereof, a spacing between the axes of the two housings being equal or slightly less than the sum of the radii of the bar and the spherical head.

11. A system according to one of claims 9 and 10, **characterized in that** it comprises a screw (23), preferably without a head and of countersink type, capable of co-operating with a female screwthread (19) of the hollow support (10) and having a hollow operating recess (24) which is for example polygonal and in particular hexagonal, to permit immobilisation of the spherical head of the anchoring member in the second housing (20).

12. A system according to claim 11 **characterized in that** the screw (23) is hollow therethrough to permit access for a tool to an operating profile (22) provided in the spherical head of the anchoring member.

13. A system according to one of claims 1 to 12, **characterized in that** the nut (9) co-operates directly with the bar (1).

14. A system according to one of claims 9 and 10, **characterized in that** it comprises an intermediate part (28) formed by a central portion (29) for bearing against the spherical head (4) of the anchoring member and intended to be received in a smooth bore (19') of the hollow support (10'), and two arms (30) passing through the wall of the hollow support by way of two longitudinal slots (32) therein, which are preferably opposite and aligned, one of the free ends of the arms (30) which are external to the hollow support being intended to come to bear against the bar (1), the assembly being such that the nut (9) bears against the free ends of the two arms (30), urging the intermediate part (28) simultaneously against the spherical head (4) of the anchoring member by way of its central portion (29) and against the bar (1) by way of the free end of one of its two arms (30).

15. A system according to claim 14 **characterized in that** the central portion (29) of the intermediate part (28) has an opening (31), the edge of which is intended to come to bear along a circular configuration against the spherical head of the anchoring member and which permits access for a tool to an operating profile provided in said spherical head.

16. A system according to one of claims 1 to 8, **characterized in that** the second housing (20) is also disposed to the exterior of the screwthreaded portion (11, 12) of the support (10") which is preferably solid in its screwthreaded portion, and is open laterally and upwardly, in opposite relationship to the rachis, so that the spherical head (4) of the anchoring member is also locked by the nut (9), on the outside of the support (10").

17. A system according to claim 16, **characterized in that** the nut (9) acts on the bar (1) and on the spherical head (4) by way of a washer (36).

18. A system according to one of claims 16 and 17, **characterized in that** the first and second housing (15, 20) are in diametrally opposite relationship with respect to the support and the nut.

19. A system according to one of claims 17 and 18, **characterized in that** the washer (36) has a window (38), the edge of which is intended to co-operate along a circular arc with the spherical head (4) and which permits access for a tool to an operating profile carried by the spherical head.

20. A system according to one of claims 1 to 19, characterized on its radial face in opposite relationship to the rachis, the nut (9) has operating recesses (27) intended to co-operate with a tightening tool.

21. A system according to one of claims 1 to 20, **characterized in that** the anchoring member belongs to the group formed by pedicellar screws (2) and hooks (3) which are for example laminar, thoracic of pedicellar.

22. A system according to one of claims 1 to 21, **characterized in that** the screwthreaded means for immobilisation of the bar (1) and the head (4) of the anchoring member are arranged to afford access from the exterior to an operating profile carried by said head, in particular by way of an opening.
